# EUROPÄISCHE PATENTSCHRIFT

(11) **EP 0 509 316 B1**
(45) Veröffentlichungstag und Bekanntmachung des Hinweises auf die Patenterteilung: **14.12.1994**
(21) Anmeldenummer: 92105591.9
(22) Anmeldetag: 01.04.1992
(51) Int. Cl.: G01N 1/00, G01N 1/22, G01N 1/28, G01N 33/18

(54) **Vorrichtung zum Messen von Fremdstoffen in einer Flüssigkeit, insbesondere Wasser**
Apparatus for determining impurities in a liquid, particularly in water
Dispositif pour mesurer des impuretés dans un liquide, notamment dans l'eau

(30) Priorität: 09.04.1991 DE 4111513; 09.04.1991 DE 4111502
(43) Veröffentlichungstag der Anmeldung: 21.10.1992
(73) Patentinhaber: Schultz, Hans-Dieter, D-22587 Hamburg (DE)
(72) Erfinder: Fuhrmann, Hans, Dr., (verstorben) (DE)
(74) Vertreter: Richter, Werdermann & Gerbaulet

(56) Entgegenhaltungen:
- EP-A- 0 256 444
- GB-A- 2 158 235
- US-A- 2 987 912
- US-A- 4 546 640

## Beschreibung

Die Erfindung betrifft eine Vorrichtung zum Messen von Fremdstoffen, insbesondere von organischen Lösungsmitteln, in einer Flüssigkeit, insbesondere Wasser, die eine mit einer Heizeinrichtung (23) versehene Misch- bzw. Reaktionskammer (16), durch die die Flüssigkeit geleitet und in der die Flüssigkeit mit einem Trägergas wie Luft, gemischt wird, wobei die Fremdstoffe zumindest teilweise in die Gasphase überführt werden, und einen Gassensor (30) zur Messung des bei der Mischung entstehenden, aus dem Trägergas und den in die Gasphase übergegangenen Fremdstoffen zusammengesetzten Gemisches umfaßt.

Aufgrund der zunehmenden Verschmutzung von Flüssen und Gewässern durch Fremdstoffe, insbesondere Kohlenwasserstoffe und halogenierte Kohlenwasserstoffe, ist eine Messung umweltschädlicher Stoffe dringend erforderlich. Dies gilt auch für die Überwachung kommunaler und industrieller Abwässer.

Bei den zu erfassenden Verschmutzungen handelt es sich in der Regel um organische Verschmutzungen, und zwar vorwiegend um im Wasser gelöste Kohlenwasserstoffe, halogenierte Kohlenwasserstoffe sowie toxische Zerfallsprodukte auf der Basis der genannten Verschmutzungen.

Hierbei ist eine kontinuierliche Messung von Vorteil, so daß eine ständige Überwachung gegeben ist und einerseits auch sehr kurzzeitige Verschmutzungen zuverlässig erfaßt werden und andererseits jeweils sehr rasch nach Auftreten einer Verunreinigung reagiert werden kann.

Probleme bereitet bei einer solchen Überwachung auch die Tatsache, daß sehr geringe organische Verschmutzungen im ppb- und ppm-Bereich zuverlässig erfaßt werden müssen, was an die Meßtechnik hohe Anforderungen stellt.

Es sind bereits Meßverfahren bekannt, bei denen die Messung durch Absorption und Desorption der Proben mit Hilfe besonderer Filtermassen und anschließender fotometrischer oder potentiometrischer Auswertung durchgeführt wird. Dies erfordert aber nachteiligerweise manuelle Eingriffe und läßt sich nur diskontinuierlich durchführen. Alternativ werden teilweise auch Gaschromatographen eingesetzt, die hohe Empfindlichkeit und Selektivität besitzen. Allerdings erlaubt auch die Gaschromatographie keine kontinuierliche Flüssigkeitsüberwachung. Zudem ist hier der Serviceaufwand ebenfalls sehr hoch, insbesondere auch aufgrund der Verwendung eines Hilfsgases (Wasserstoff).

Eine Vorrichtung zur kontinuierlichen Messung organischer Lösemittel in einer Flüssigkeit ist aus der DE-A-31 26 648 bekannt. Dort wird ein Halbleitergassensor direkt in die Flüssigkeit eingetaucht, der aber durch ein flüssigkeitsundurchlässiges Medium von der Flüssigkeit getrennt ist. Das flüssigkeitsundurchlässige Medium erlaubt eine Diffusion der gelösten freien Gase aus der Flüssigkeit zum Halbleitergassensor, so daß dessen Ausgangssignal repräsentativ für den Anteil organischer Lösemittel in der Flüssigkeit ist. Diese Vorrichtung erfordert allerdings eine Direkteintauchung des Gassensors in die Flüssigkeit mit entsprechenden Abdichtungsproblemen. Zudem kann bei schwankenden Flüssigkeitsspiegeln eine Lageregelung des Sensors erforderlich werden, um sicherzustellen, daß dieser stets in die Flüssigkeit eintaucht, jedoch nicht vollständig in dieser untergetaucht ist. Auch die Anforderungen hinsichtlich des flüssigkeitsundurchlässigen, den Gasdurchtritt erlaubenden Mediums sind verhältnismäßig hoch.

Ferner ist es aus der DE-A-32 21 063 bekannt, eine Flüssigkeit durch Zusatz von Reagenzien zur Erzeugung einer chemischen Farbreaktion unter Einsatz einer fotometrischen Meßeinrichtung zu überwachen. Das Reaktionsgefäß ist dort als senkrecht stehender Zylinder ausgebildet, in den unterseitig die zu überwachende Flüssigkeit eingeführt wird. In das Reaktionsgefäß münden weiterhin Zuführungsleitungen, über die die verschiedenen Reagenzien gesteuert eingeleitet werden können. Die bekannte Apparatur erfordert einen höheren konstruktiven Aufwand und genaue Dosierung. Zudem muß der Flüssigkeitsstrom während einer jeweiligen Messung unterbrochen werden, so daß keine vollständig kontinuierliche Flüssigkeitsüberwachung möglich ist.

Durch die GB-A-2 158 235 ist ein Kontrollgerät für im Wasser enthaltene Schadstoffe bekannt, bei dem das dem Gerät zugeführte Probenwasser vermittels einer Heizeinrichtung erwärmt und durch eine Mischkammer geleitet wird, der ein Gas zugeführt wird, um die im Probenwasser flüchtigen Schadstoffe abzuführen und einem Meßgerät zuzuführen, das auf das Vorhandensein und/oder auf die Konzentration des Schadstoffes in dem Gas anspricht.

Eine Einrichtung zur Überwachung leichtflüchtiger chlorierter und fluorierter Kohlenwasserstoffe in Abwässern mit einem Meßgerät beschreibt die EP-A-0 256 444. Bei dieser Überwachungseinrichtung ist an der Gaseintrittsseite des Gasmeßgerätes eine Entnahmevorrichtung mit einem Phasentauscher angeschlossen. Eine Luftleitung ragt in den vom Abwasser durchflossenen Phasentauscher hinein, durch die Luft zum Austreiben der im Abwasser nachzuweisenden Kohlenwasserstoffe gedrückt wird. Am Umfang des Phasentauschers sind Öffnungen verteilt angeordnet, so daß durch teilweisen Austritt der Luft aus den Öffnungen fortwährend frisches Meßmedium an der Unterseite des Phasentauschers angesaugt und nach Phasentausch aus dieser Öffnung wieder herausgeführt wird. Eine weitere Luftleitung ist an einer Austrittsseite des Phasentauschers angeschlossen, durch die die ausgetriebenen Kohlenwasserstoffanteile in einem Luftstrom als Meßluft dem Gasmeßgerät zugeführt werden. Mit dieser Einrichtung sind leichtflüchtige chlorierte und fluorierte Kohlenwasserstoffe in Abwässern überwachbar, wobei als Überwachungsgerät ein Gasmeßgerät verwendet wird. Ein zur Überwachung von Schadstoffen in der Umgebungsluft geeignetes Meßgerät kann somit zur Überwachung von Schadstoffen in Abwässern eingesetzt werden.

Die US-A-2 987 912 beschreibt ein Verfahren zur Messung der Menge eines in einer Flüssigkeit gelösten Gases, das das Spülen eines Behälters in einem geschlossenen System mit einem neutralen Gas einschließlich des Geräts zur Messung der Menge des gelösten Gases, weiterhin das Füllen des Behälters mit der Flüssigkeit bis zu einem vorher bestimmten Niveau, um ein gleichbleibendes Verhältnis von Gas zur Flüssigkeit herzustellen, während gleichzeitig das neutrale Gas im System zurückgehalten wird und das neutrale Gas in hochaufgelöstem Zustand durch die Flüssigkeit zirkuliert, um das aufgelöste Gas aus der Flüssigkeit abzuscheiden und die Veränderung des Geräts nach einem vorher bestimmten Zirkulationszeitraum, der wesentlich kürzer ist, als der zum Erreichen des Gleichgewichts zwischen dem in der Flüssigkeit aufgelösten Gas und dem abgeschiedenen Gas, welches die Menge des gelösten Gases in der Flüssigkeit anzeigt, umfaßt. Die zur Durchführung dieses Meßverfahrens eingesetzte Vorrichtung umfaßt einen geschlossenen Probenbehälter mit Geräten zum Einbringen und Entfernen der zu prüfenden Flüssigkeit, ein Gaszirkulationssystem und einem Abzug aus dem freien Raum oberhalb der Probe in dem Probenbehäter, ein Gerät, das operativ mit diesem Abzug verbunden ist, um die Menge des gelösten Gases, welches sich in dem im Abzug zirkulierenden Gas befindet, zu messen, einen Abzug vom Meßgerät zur Rückführung des zirkuliernden Gases in die Flüssigkeitsprobe, die sich unten im Behälter befindet, ein Seitenabzug, der mit einem der anderen Abzüge zum Ausstoß des Gases in die Atmosphäre verbunden ist, ein Gaspumpgerät, das mit einem der Abzüge zur Zirkulation des Gases aus dem Behälter durch das System verbunden ist, einen Abzug, der mit dem System zum Durchlaß eines neutralen Gases durch das System und den Behälter verbunden ist und im Abzug eingebaute Ventile.

Die US-A-4 546 640 beschreibt ein Gasspürgerät zum Messen der in einem Becken mit stark dickflüssigem Stoff vorhandenen Gasmenge, das einen nach unten offenen sowie nach oben geschlossenen Behälter aufweist, der sich in dem Becken befindet und dessen untere Wände unterhalb der Oberfläche des dickflüssigen Stoffes und vom Beckenboden entfernt liegen, so daß der Stoff sich frei vom Becken in den Behälter bewegen kann, wobei das obere Ende des Behälters sich hierbei über der Oberfläche befindet und so eine Luftkammer über der Oberfläche des Beckenmaterials bildet. Ein Einlaßrohr ist mit einer Abflußröhre versehen, die sich im Behälter unterhalb der Oberfläche des dickflüssigen Stoffes befindet. Mit der Einlaßöffnung des Rohres ist ein Gleichdruckgerät zur Erzeugung einer Bewegung des Luftstroms im dickflüssigen Stoffes im Behälter verbunden, um so das Restgas daraus zu entfernen, wobei das Gas und die Druckluft aufsteigen und sich in der Luftkammer bei höherem Druck als der Luftdruck sammeln. Des weiteren ist mit der Luftkammer ein durch eine erste Entlüftungsöffnung verbundenes Auslaßrohr verbunden, wodurch das Druckgas und die in der Luftkammer befindliche Luft durch die Entlüftungsöffnung und das Auslaßrohr ausgestoßen werden. Ein Durchflußmeßgerät ist nach unten mit dem Auslaßrohr verbunden, um somit das in der Luftprobe der Luftkammer vorhandene Gas wahrzunehmen. Dieses Gasspürgerät ist zum Auffinden von Gas in einer viskosen Flüssigkeit vorgesehen, wobei Druckluft in die viskose Flüssigkeit eingebracht wird, um diese in Bewegung zu versetzen und um das Gas zu entfernen. Das so entfernte Gas wird zusammen mit der Druckluft in dem verschlossenen Behälter aufgefangen. Aus diesem Behälter wird über einen Auslaß vermittels Druckluft das Gas herausgedrückt, das dann dem Meßsystem zugeführt wird.

Bei diesen Meßgeräten ist jedoch keine Kalibrierung bzw. Eichung vorgesehen, um sicherzustellen, daß keine oder nur vernachlässigbar geringe Fremdstoffkonzentration im Gas vorhanden ist, so daß der Sensor nur reines Trägergas mißt und sein Ausgangssignal als Nullwert kalibriert werden kann. Auch ist ein Erfassen von organischen Lösungsmitteln im Wasser nicht möglich.

Der Erfindung liegt die Aufgabe zugrunde, eine Vorrichtung zum Messen von Fremdstoffen in einer Flüssigkeit, insbesondere Wasser, zu schaffen, die eine Erfassung auch sehr geringer Fremdstoffkonzentrationen im ppb- oder ppm-Bereich bei verhältnismäßig einfachem Aufbau ermöglicht. Insbesondere soll eine stabile und kontinuierliche Überwachung von organischen Lösungsmitteln in Wasser möglich sein.

Diese Aufgabe wird mit den in dem Patentanspruch 1 angegebenen Merkmalen gelöst.

Vorteilhafte Ausgestaltungen der Erfindung sind in den Unteransprüchen angegeben.

Bei der Vorrichtung nach der Erfindung wird die Flüssigkeit mit einem Trägergas gemischt, so daß die in der Flüssigkeit gelösten Fremdstoffe zumindest teilweise in die Gasphase überführt werden. Das aus dem Trägergas und den Fremdstoff-Gasen zusammengesetzte Gasgemisch wird mittels eines Gassensors gemessen. Der Gassensor muß folglich nicht in direkten Kontakt mit der Flüssigkeit gebracht werden, so daß keine aufwendige Flüssigkeitsabschirmung des Sensors erforderlich ist. Dies vereinfacht den Aufbau. Die Durchmischung der Flüssigkeit mit dem Trägergas kann kontinuierlich, bei strömender Flüssigkeit, erfolgen, so daß eine kontinuierliche Flüssigkeitsüberwachung erreichbar - ist. Selbstverständlich ist es aber auch möglich, die Messung diskontinuierlich durchzuführen, wenn beispielsweise lediglich Stichproben gewünscht werden oder der Flüssigkeitsstrom nur diskontinuierlich anfällt, wie bei manchen Abwässern.

Die Durchmischung der Flüssigkeit mit dem Trägergas erfolgt durch einfache Einleitung des Trägergases in den Flüssigkeitsströmungspfad, so daß der apparative Aufwand sehr gering ist.

An die Mischkammer schließt sich eine Trennkammer an, in der die Flüssigkeit vom Gasgemisch getrennt wird. Diese Trennung wird in äußerst einfacher Weise dadurch realisiert, daß die Trennkammer eine größere Höhenerstreckung aufweist, so daß sich die Flüssigkeit im unteren Bereich sammelt, während das Gasgemisch nach oben steigt. Die Mischkammer ist in diesem Fall als enge Röhre ausgebildet, die eine sehr effektive Vermischung von Flüssigkeit und Trägergas erlaubt. Damit können die gelösten Fremdstoffgase wirksam in das Trägergas übertreten, so daß verhältnismäßig hohe Fremdgaskonzentrationen im Trägergas, die zuverlässig erfaßbar und auswertbar sind, erzielt werden.

Der Gassensor ist dabei im oberen Bereich der Trennkammer angeordnet, so daß eine Benetzung durch Flüssigkeit vermieden wird und das zu messende Gasgemisch selbsttätig zum Gassensor strömt.

Zum Ableiten der Flüssigkeit ist die Trennkammer mit einem Ablauf versehen. Die Flüssigkeit kann intermittierend abgeführt werden, wird jedoch bei kontinuierlicher Überwachung bevorzugt kontinuierlich abgeleitet, so daß das jeweilige Flüssigkeitsvolumen in der Trennkammer und damit auch das Gasvolumen im wesentlichen konstant bleiben und die Gasmessung demzufolge bei konstantem Gasvolumen und Druck exakt stattfinden kann.

Im Ablauf ist eine Überlaufkappe vorhanden, die als flexibler Verschluß dient, so daß die Flüssigkeit kontinuierlich ablaufen kann, jedoch kein Gas über den Ablauf austreten kann. Das Gasgemisch kann folglich nur unter Vorbeiströmen am Gassensor über im Sensorbereich vorhandene Austrittsöffnungen entweichen. Dies erlaubt eine exakte Messung.

Im Bereich der Mischkammer ist eine Heizeinrichtung vorgesehen, so daß die Temperatur in der Mischkammer im wesentlichen konstant gehalten wird. Damit kann der Gasdruck in der Mischkammer und somit die Rate des Übertretens der gelösten Fremdstoffe, insbesondere der organischen Lösemittel, in die Gasphase standardisiert werden, so daß eine jeweilige Fremdstoffkonzentration zu einem temperaturunbeeinflußten eindeutigen Meßwert des Gassensors führt. Dies gewährleistet hohe Meßgenauigkeit.

Die Heizeinrichtung kann außenseitig der Mischkammer oder in der Mischkammer-Wandung angeordnet sein. Bevorzugt ist sie jedoch im Innern der Mischkammer angebracht und befindet sich in direktem Kontakt mit der Flüssigkeit, so daß ein unmittelbarer, effektiver und rascher Wärmeübergang auf die Flüssigkeit sichergestellt wird. Dies erlaubt eine schnelle Temperaturregelung. Ferner sind Heizungsverluste aufgrund Wärmeabstrahlung nach außen u.dgl. äußerst gering. Die Heizung kann somit relativ klein ausgelegt sein.

Um die Flüssigkeitstemperatur zu erfassen, ist in bevorzugter Ausgestaltung ein Temperaturdetektor vorhanden. Das Temperatursignal des Temperaturdetektors kann zur Temperaturregelung der Flüssigkeitstemperatur und/oder als Korrektursignal zur Zuordnung des vom Gassensor abgegebenen Meßsignals zu einer bestimmten Fremdstoffkonzentration, in Abhängigkeit vom Temperaturkennfeld, eingesetzt werden.

Für eine Kalibrierung/Eichung ist es vorteilhaft, das Trägergas, das vorzugsweise aus lösemittelfreier Luft besteht, direkt zum Gassensor zu führen. Bei Durchführung einer Messung in diesem Zustand ist sichergestellt, daß keine, oder nur vernachlässigbar geringe, Fremdstoffkonzentrationen im Gas vorhanden sind, so daß der Sensor reines Trägergas mißt und sein Ausgangssignal als Null-Wert kalibriert werden kann.

In der Trägergas-Zuführleitung befindet sich ein Ventil, durch das das Trägergas entweder auf die Bypass-Leitung zum Gassensor oder auf eine zur Mischkammer führende Leitung umgeschaltet werden kann. Der Kalibriervorgang kann dabei durch einfache Ventilumschaltung auf die Bypass-Leitung eingeleitet werden.

Um den Kalibriervorgang in seiner Genauigkeit noch weiter zu erhöhen, wird dieser mit einer Standardlösung durchgeführt . Um dies zu ermöglichen, ist vorzugsweise in der Flüssigkeitszuleitung ein Ventil angeordnet, das auf die Förderung der Standardlösung zur Mischkammer umschaltbar ist. Der normale Flüssigkeitsstrom wird somit gesperrt und durch die Standardlösung ersetzt. Folglich liegen während der Kalibrierung exakt definierte Verhältnisse vor.

Als Trägergas kann jedes Gas zum Einsatz kommen, sofern sichergestellt ist, daß es frei von den zu messenden Fremdstoffen ist. In besonders einfacher und effektiver Ausgestaltung wird als Trägergas Luft verwendet, die über ein Filter, vorzugsweise ein Aktivkohlefilter, angesaugt wird. Damit ist kein eigener Trägergas-Vorrat erforderlich, was der Einfachheit und Robustheit sowie Ortsungebundenheit des erfindungsgemäßen Meßgeräts entgegenkommt.

Die Meßgenauigkeit kann noch weiter dadurch erhöht werden, daß sowohl das Trägergas als auch die Flüssigkeit in bestimmtem, festem Verhältnis in die Mischkammer eingeleitet werden. Dies erfolgt vorzugsweise durch synchronen Betrieb von Pumpen, die das Trägergas und die Flüssigkeit fördern. Steuerungstechnisch besonders einfach ist es, die Pumpen mechanisch miteinander zu koppeln.

Die Meßgenauigkeit kann noch weiter gefördert werden, wenn der Gassensor mit einer Heizeinrichtung versehen ist. Damit können definierte Temperaturverhältnisse im Bereich des Gassensors eingestellt werden, so daß dessen Meßsignal nicht durch Temperatur-Drifteffekte oder dergleichen verfälscht wird. Vorzugsweise wird die Temperatur so geregelt, daß sie oberhalb der Temperatur des zu messenden Gasgemisches liegt. Hierdurch kann vermieden werden, daß das Gasgemisch im Bereich des Gassensors kondensiert und damit die Meßergebnisse verfälscht.

Die Mischkammer ist als langgestrecktes, im wesentlichen horizontal verlaufendes Rohr ausgebildet. Hierbei wird effektive Durchmischung der Flüssigkeit mit dem Trägergas erreicht, so daß das in der Flüssigkeit enthaltene gelöste Fremdstoffgas in das Trägergas überdiffundieren, d.h. in die Gasphase übertreten kann. Die Rohrlänge kann beispielsweise 100 mm bei einem Innendurchmesser von 3 mm betragen.

Eine derartige Vorrichtung erlaubt eine zuverlässige und exakte Messung von Fremdstoffen bei verhältnismäßig geringem Aufwand. Als Fremdstoffe können all diejenigen Stoffe überwacht werden, die bei Durchmischung der Flüssigkeit mit Trägergas in die Gasphase überführt werden können. Insbesondere ermöglicht die Erfindung aber eine stabile und auch kontinuierliche überwachung von organischen Lösungsmitteln in Wasser, wobei als Trägergas in sehr einfacher Weise Luft verwendet werden kann. Die Erfindung erlaubt die Erfassung auch äußerst geringer Konzentrationen im ppb- oder ppm-Bereich.

Die Genauigkeit und Reproduzierbarkeit der Meßergebnisse läßt sich noch dadurch weiter erhöhen, daß die Flüssigkeit und das Trägergas in vorbestimmtem gegenseitigem Fördermengenverhältnis zur Reaktionskammer geführt werden. Die Fördermengen können hierbei jeweils auf konstante Werte eingestellt werden. Aber auch dann, wenn variable Fördermengen, beispielsweise aufgrund un-terschiedlichen Flüssigkeitsanfalls, eingestellt werden, ist dennoch sichergestellt, daß das Mischverhältnis stets konstant ist, so daß auch die Übertrittsrate der Fremdstoffe in das Trägergas konstant bleibt. Das Meßergebnis wird damit von der jeweiligen Fördermenge im wesentlichen unabhängig.

Als Trägergas wird Luft verwendet, die über ein Filter angesaugt wird. Damit ist für diese Vorrichtung kein spezieller Trägergasvorrat erforderlich, was die Einfachheit und Robustheit des Aufbaus fördert. Zudem ist die erfindungsgemäße Vorrichtung universell überall dort einsetzbar, wo Flüssigkeit auf Fremdstoffe zu überwachen ist.

Um auch verhältnismäßig warme Flüssigkeiten messen zu können, kann eine zusätzliche Kühleinrichtung vorhanden sein, über die die Flüssigkeit vor ihrer Einleitung in die Reaktionskammer gekühlt wird. Damit läßt sich die Flüssigkeit auf definierte Temperaturwerte einstellen, so daß reproduzierbare Meßergebnisse erzielt werden.

Eine derart ausgebildete Vorrichtung erlaubt eine sehr einfache und dennoch effektive Messung von Fremdstoffen, wie etwa organischen Verschmutzungen, bei denen es sich vorwiegend um im Wasser gelöste Kohlenwasserstoffe, halogenierte Kohlenwasserstoffe oder toxische Zerfallsprodukte auf der Basis solcher Verschmutzungen handelt. Hierbei wird die im Wasser gelöste Meßkomponente, wie etwa Per- oder Trichlorethylen, Methylenchlorid und andere, sowie nicht-halogenierte Kohlenwasserstoffe, in die Gasphase überführt. Als Trägergas wird in diesem Fall lösemittelfreie Luft verwendet. Die Erfindung erlaubt auch die Erfassung äußerst niedriger Konzentration im ppb- und ppm-Bereich. Die Arbeitsweise der erfindungsgemäßen Vorrichtung kann als Phasenaustauschverfahren klassifiziert werden.

Die Spurenmessung der in die Gasphase überführten Fremdstoffe erfolgt dabei auf physikalischer Basis, mit Hilfe des Gassensors.

Die Vorrichtung ist insbesondere im praktischen Einsatz im Rahmen des Umweltschutzes gut geeignet und erlaubt eine kontinuierliche Messung bestimmter Gruppen- und Summenparameter von Fremdstoffen, insbesondere organischen Lösemitteln, in Wasser. So können beispielsweise für die Trinkwasseraufbereitung aus Uferfiltraten kurzoder langzeitige Störungen im Rohwasser, z. B. bei organischer Verschmutzung des Flußwassers, rechtzeitig erkannt und entsprechende Schritte unternommen werden. Auch bei Arbeitsunfällen, z. B. Leckagen von Tanks, Überlaufen von Tankwagen und sickern der Schadstoffe in das Erdreich kann eine mögliche Ausbreitung im Grundwasser rechtzeitig bemerkt werden. Dies gilt sowohl für ambulante als auch stationäre Anlagen, z. B. bei Grundwasser-Untersuchungen unterhalb industrieller Anlagen, in denen organische Lösemittel verarbeitet werden, oder auch bei der überwachung des Grundwassers unterhalb eines Flufhafengeländes.

Die Vorrichtung kann "rund um die Uhr" betrieben werden und bietet hohe Verfügbarkeit bei geringem Wartungsaufwand. Durch die Eingrenzung spezifischer Komponenten mit Summen- und Gruppenparametern lassen sich die Fremdstoffe, insbesondere organische Lösemittel, sehr einfach und dennoch genau messen.

Die Erfindung wird nachstehend anhand von Ausführungsbeispielen unter Bezugnahme auf die Zeichnungen näher beschrieben. Es zeigen
Fig. 1 eine schematische Darstellung eines Ausführungsbeispiels der erfindungsgemäßen Vorrichtung bei Durchführung eines Meßvorgangs,
Fig. 2 das Ausführungsbeispiel gemäß Fig. 1 bei Durchführung der Nullpunkt-Einstellung,
Fig. 3 mehrere Kalibrierkurven,
Fig. 4 Temperatur- und Widerstands-Kennlinien,
Fig. 5 ein Meßprotokoll bei der Messung von Methylenchlorid,
Fig. 6 ein Schaltbild der Temperatursteuerung der Mischkammer und des Gas-Sensors und
Fig. 7 und 8 weitere Meßkurven.

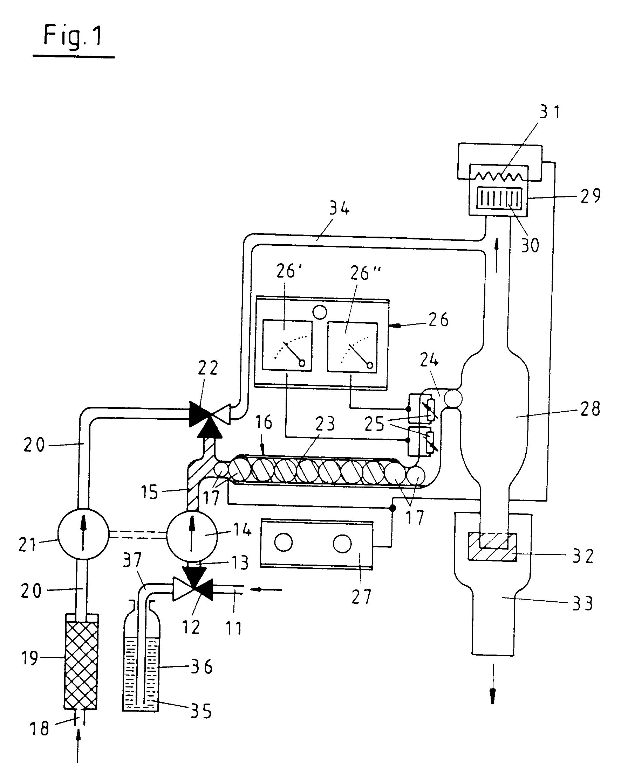

In Figur 1 ist schematisch ein Ausführungsbeispiel der erfindungsgemäßen Vorrichtung dargestellt, und zwar in Form eines Fließbilds während der Fremdstoffmessung. Die zu überwachende Flüssigkeit, vorzugsweise Wasser, wird über eine Leitung 11 zugeführt, in der ein als Drei-Wege-Ventil ausgebildetes Ventil 12 angeordnet ist. Bei Durchführung einer Fremdstoffüberwachung ist das Ventil 12 in der dargestellten Weise so geschaltet, daß es die Leitung 11 mit einer Leitung 13 verbindet, so daß die Flüssigkeit in die Leitung 13 strömt.

In der Leitung 13 sitzt eine Pumpe 14 zur Förderung der Flüssigkeit, an die ausgangsseitig ein T-förmiges Leitungsstück 15 anschließt. Der horizontale Ast des T-förmigen Leitungsstücks 15 mündet in eine Mischkammer 16. Die Mischkammer 16 hat beispielsweise eine Länge von 100 mm bei einem Innendurchmesser von 3 mm und einem Außendurchmesser von 7 mm. Das Volumen der Mischkammer 16 beträgt somit ca. 0,7 ml. Das Fördervolumen der Pumpe 14 ist so ausgelegt, daß z.B. ein Flüssigkeitdurchsatz von 500 ml/h durch die Mischkammer 16 erreicht wird.

In der Mischkammer 16 wird die Flüssigkeit mit Trägergas gemischt, das durch die Gasblasen 17 veranschaulicht ist. Das Trägergas wird durch Ansaugung von Luft über eine Einlaßöffnung 18 gewonnen, die in eine Filterkammer 19 mündet, in der sich ein Aktivkohlefilter befindet. Am Auslaß der Filterkammer 19 ist eine Leitung 20 angeordnet, in die eine Pumpe 21 zur Ansaugung und Weiterförderung der Luft geschaltet ist. Die Leitung 20 mündet in ein Ventil 22, das als Drei-Wege-Ventil ausgebildet ist und in der dargestellten Fließschaltung die Luft von der Leitung 20 auf das T-förmige Leitungsstück 15 weiterleitet. Das Ventil 22 ist ebenso wie das Ventil 12 als Magnetventil ausgebildet, d.h. elektrisch steuerbar.

Die Pumpen 14 und 21 werden bei der Fremdstoffmessung so betrieben, daß das Verhältnis zwischen geförderter Flüssigkeit und gefördertem Trägergas konstant ist. Um dies zu erreichen, sind die Pumpen 14 und 21 mechanisch gekoppelt, so daß das Verhältnis ihrer Förderleistungen selbst bei Veränderungen der absoluten Fördermenge jeweils konstant bleibt. Die Pumpen 14 und 21 dienen als Dosiereinheiten und können auch durch andere geeignete Fördereinrichtungen ersetzt werden.

In der Mischkammer 16 diffundieren die in der Lösung befindlichen Spuren des Meßgases, d.h. der zu messenden Fremdstoffe, innerhalb der Verweilzeit der Flüssigkeitsprobe in der Mischkammer 16 in das Trägergas. Diese Diffusion wird durch die Trägergasblasen in der Mischkammer, die in der Flüssigkeit aufgrund der Strömung verwirbelt werden, in sehr effektiver Weise gefördert, so daß die Fremdstoffe, vorzugsweise die gelösten organischen Lösemittel, in verhältnismäßig hohem Umfang in die Gasphase überwechseln und sich mit dem Trägergas mischen. Die Flüssigkeit wird folglich entgast. Zur Temperatureinstellung befindet sich in der Mischkammer 16 eine nachstehend noch näher beschriebene Heizeinrichtung 23.

Die Mischkammer 16 geht über ein Leitungszwischenstück 24, in dem sich mit einer Anzeige- und Regeleinrichtung 26 und einer Spannungsversorgungseinrichtung 27 verbundene Meßwiderstände 25 befinden, in eine Trennkammer 28 über, die als Phasenaustauschkammer dient. Die Trennkammer 28 bewirkt eine Trennung des aus der Mischkammer 16 kommenden Flüssigkeits-/Gasblasen-Gemischs in die entgaste, nach unten sinkende Flüssigkeit und das aus Trägergas und in die Gasphase übergegangenem Lösemittelgas bestehende Gasgemisch, das nach oben steigt. Die Trennkammer 28 erstreckt sich in vertikaler Richtung und besitzt im Einmündungsbereich des Leitungszwischenstücks 24 einen verbreiterten Abschnitt, an den sich nach oben und unten dünnere Abschnitte anschließen. Der oberseitige querschnittsverringerte Abschnitt führt zu einem vertikal oben sitzenden Gassensor 30, der vorzugsweise in einer Gas-Meßkammer 29 angeordnet ist. Zur Temperatureinstellung des Gassensors 30 und der Meßkammer 29 ist eine Heizeinrichtung 31 vorhanden.

Der unterseitige querschnittsverjüngte Abschnitt der Trennkammer 28 mündet in eine Überlaufkappe 32, die in einem Auslauf 33 angeordnet ist. Die Überlaufkappe 32 dient als flexibler Verschluß für die ablaufende, entgaste Flüssigkeit, so daß das aus der Probe diffundierte Meßgas nicht entweichen kann. Die überlaufkappe 32 ist so angeordnet, daß ihr Flüssigkeitsspiegel oberhalb der unteren Austrittsöffnung der Trennkammer 28 liegt.

Das aus der Probelösung ausgetretene Meßgas (Gasgemisch) wird in der Gas-Meßkammer 29 gemessen, die durch die Heizeinrichtung 31 temperiert wird. Die Heizeinrichtung ist als elektrisch angesteuerte Heizwicklung ausgebildet. Die Temperatur in der Gas-Meßkammer 29 wird thermostatisch so geregelt, daß die Umgebungstemperatur im Inneren der kleinen Meßkammer ca. 3° C oberhalb der Temperatur des Meßgases, d.h. des Gasgemisches, liegt, um eine Kondensation zu vermeiden.

Die Temperatur der Flüssigkeit, d.h. der Probelösung, wird über die beiden Widerstandsthermometer 25 gemessen und auf die eingestellte Wassertemperatur der Probe geregelt. Die entsprechenden Bauelemente befinden sich in der Anzeige- und Regeleinrichtung 26, die mit zwei Anzeigeeinrichtungen 26′, 26′′ ausgestattet ist. über eine der Anzeigeeinrichtungen kann die eingestellte Soll-Temperatur und über die andere die Ist-Temperatur der Flüssigkeit angezeigt werden. Die Temperaturregelung der Flüssigkeit erfolgt unter Einsatz der Heizeinrichtung 23, die als Heizspirale im Inneren der Mischkammer 16 ausgebildet ist und durch die Spannungsversorgungseinrichtung 27 mit Spannung gespeist wird. Die Spannungsregelung und/oder Ein-/Ausschaltung der SpannungsVersorgung der Heizeinrichtung 23 wird über in der Anzeige- und Regeleinrichtung 26 angeordnete, nicht gezeigte Regelkomponenten so gesteuert, daß die Flüssigkeit in der Mischkammer die gewünschte Temperatur annimmt. Die Spannungsversorgungsschaltung 27 versorgt auch die Heizeinrichtung 31 mit Strom.

Die Spannungsversorgungseinrichtung 27 kann als Regler ausgebildet sein, durch den die Stromversorgung für die Heizeinrichtung 23 und die Heizeinrichtung 31 konstant auf einem vorprogrammierten Strom gehalten wird. Die einzuregelnden Temperaturen sind auf Werte von 15 bis 40°C einstellbar.

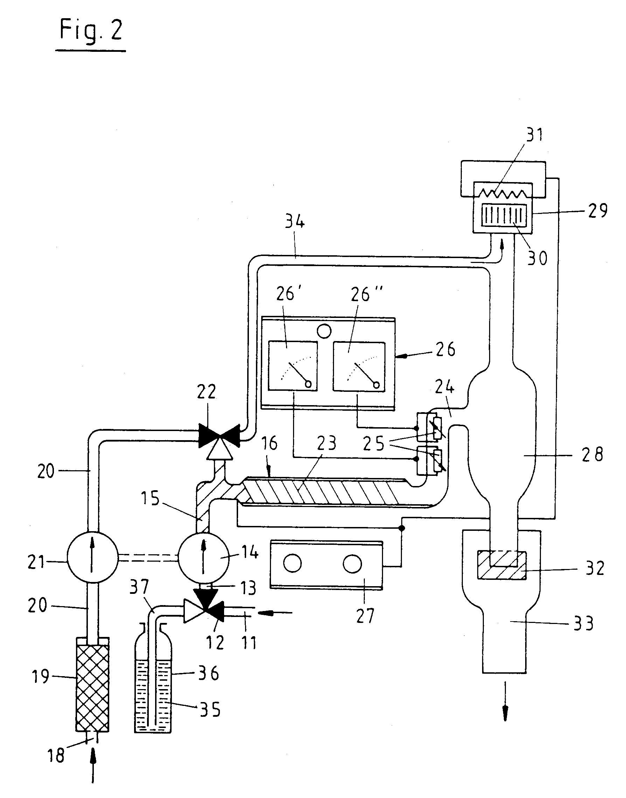

In Figur 2 ist das Ausführungsbeispiel gemäß Figur 1 in der Ventilstellung für Nullpunkt-Einstellung gezeigt. Für die Durchführung der Nullpunkt-Einstellung ist lediglich eine Umschaltung des Ventils 22 erforderlich, und zwar auf Durchgang von der Leitung 20 zu einer By-pass-Leitung 34, die vom Ventil 22 ausgeht und unter Umgehung der Mischkammer 16 direkt in die Trennkammer 28 einmündet, und zwar vorzugsweise in deren oberem Ab-schnitt direkt unterhalb des Gas-Meßraums 29. Die Umstellung von normaler Messung auf Nullpunkt-Einstellung ist somit in äußerst einfacher Weise lediglich durch Umschaltung eines einzigen Ventils möglich. Bei der Nullpunkt-Einstellung wird die Probenflüssigkeit unverändert über den Einlaß 11, das Ventil 12 und die Pumpe 14 zur Mischkammer 16 geführt. Das Trägergas, d.h. vorzugsweise die gereinigte Luft, gelangt jedoch aufgrund der Umschaltung des Ventils 22 nun nicht mehr in die Mischkammer 16, so daß die in Figur 1 gezeigte Blasenbildung entfällt. Damit können nur noch sehr minimale Mengen an Gaspartikeln aus der Mischkammer 16 herausdiffundieren. Folglich nimmt die Fremdstoffkonzentration in der Trennkammer 28 stark ab. Das Trägergas, d.h. die durch das Aktivkohlefilter gereinigte lösungsmittelfreie Luft, gelangt nun über die Bypass-Leitung 34 direkt in die Trennkammer 28 und zum Gas-Meßraum 29. Dort verdrängt sie eventuell noch vorhandene, aus dem Sensorgehäuse kommende Gaspartikel durch die Trennkammer 28 in den Ablauf 33. Der Gassensor 30 ist somit ausschließlich Null-Gas, d.h. Nullpunkt-Luft, ausgesetzt, so daß das von ihm abgegebene Signal das Nullpunkt-Signal bildet. Hierdurch läßt sich eine zuverlässige Null-Einstellung bewirken.

Um nicht nur den Nullpunkt einstellen, sondern auch den Meßkurvenverlauf kalibrieren zu können, wird, vorzugsweise nach Einstellung des Nullpunkts in der Ventilstellung gemäß Figur 2, von der normalen Probelösung auf Standardlösung 35 umgeschaltet. Die Standardlösung 35 befindet sich in einem Behälter 36, der über eine Leitung 37 mit dem Ventil 12 verbunden ist. Zur Kalibrierung wird das Ventil 12 so geschaltet, daß die Leitung 37 mit der Leitung 13 auf Durchgang geschaltet ist, während das Ventil 22 wieder in die Stellung gemäß Figur 1 umgeschaltet wird. In dieser Stellung wird somit durch die Pumpen (Dosiereinheiten) 14 und 21 sowohl die Standardlösung als auch das Trägergas in die Mischkammer 16 eingepumpt. Die Arbeitsweise ist in diesem Fall dieselbe wie bei der Messung der normalen Probenflüssigkeit, da in der Mischkammer 16 erneut Blasen gebildet werden. Hierbei diffundieren die in der Standardlösung, die auf einen definierten Konzentrationswert eingestellt ist, enthaltenen gelösten Gase in das Trägergas über und gelangen in der Trennkammer 28 nach oben. Im Gassensor 30 herrscht demgemäß eine entsprechende Gaskonzentration, die elektronisch ausgewertet wird. Da die Gaskonzentration aufgrund der eingesetzten Standardlösung einen definierten Wert besitzt, kann dieser zur Kalibrierung der Meßwerte eingesetzt werden.

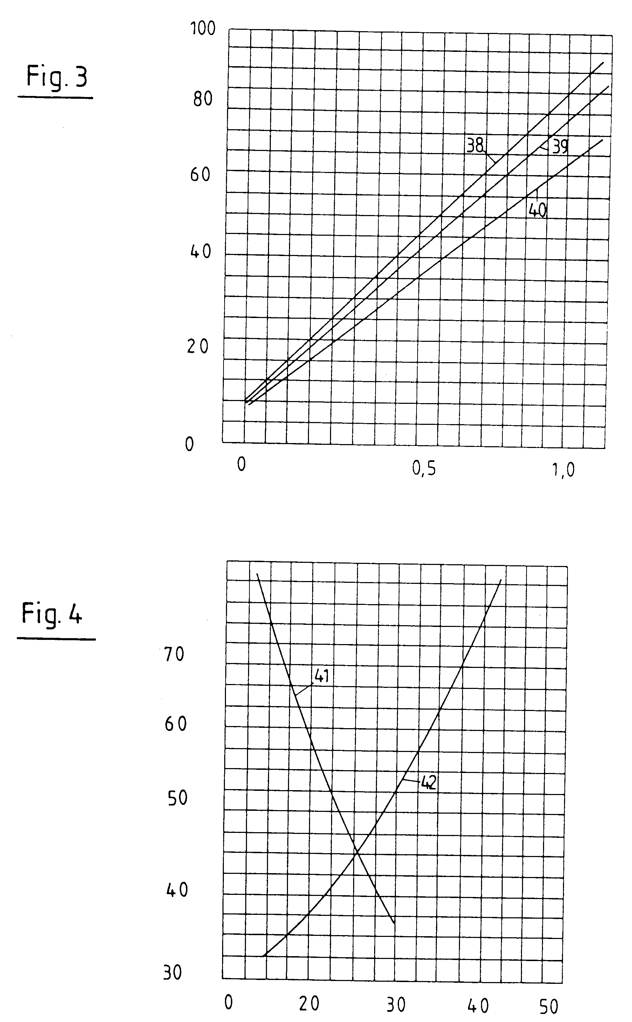

In Figur 3 sind die Kalibrierkurven für drei unterschiedliche Stoffe gezeigt. Auf der Abszisse ist die Konzentration in ppm aufgetragen, während die Ordinate die Skalenteile wiedergibt. Die Kurve 38 repräsentiert die Kalibrierkurve für Tetrachlorkohlenstoff, während die Kurven 39 und 40 die Kalibrierkurven für 1,2-Dichlorethan (Kurve 39) und Methylenchlorid (Kurve 40) darstellen. Schon bei äußerst geringen Konzentrationswerten unterhalb von 1,0 ppm wird ersichtlich schon ein deutlicher Skalenausschlag erzielt, so daß eine sehr präzise Messung und Erfassung auch feinster Verunreinigungen möglich ist.

In Figur 4 veranschaulicht die Kurve 41 den Verlauf der Temperatur (in °C), wobei auf der Ordinate der Skalenausschlag, d.h. die Skalenteile aufgetragen sind. Ersichtlich ist der Skalenausschlag bei einer bestimmten Konzentration stark von der Temperatur beeinflußt. Die Temperaturregelung ermöglicht somit eine exakte Messung, bei der jeder Meßwert direkt einer bestimmten Konzentration zugeordnet werden kann.

Die Kurve 42 in Figur 4 zeigt den Verlauf des Widerstands Rᵢ.

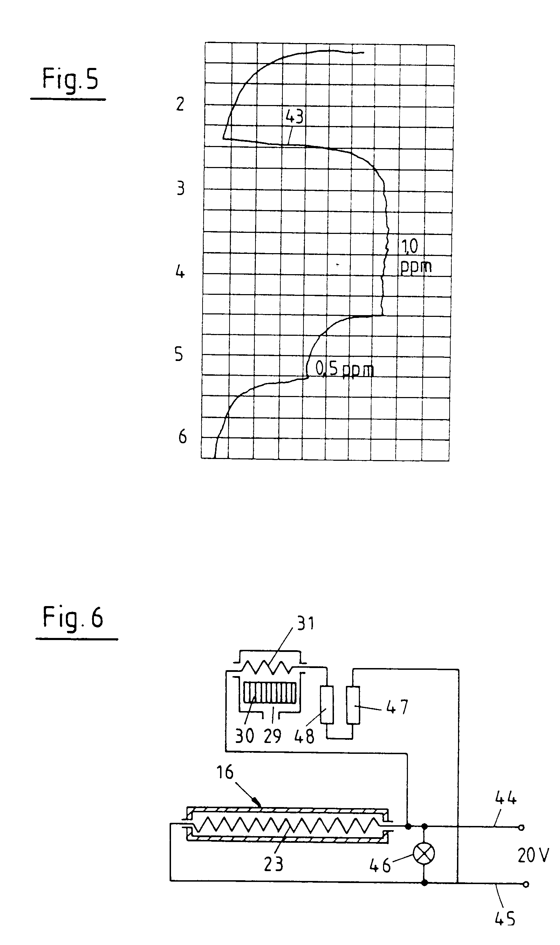

In Figur 5 ist ein bei der Messung von Methylenchlorid erzieltes Meßergebnis in Form eines Registrierstreifens dargestellt. Die erzielte Meßkurve ist mit 43 bezeichnet. Die Konzentration von Methylenchlorid wurde jeweils zwischen den Werten 0; 0,5 ppm und 1 ppm geändert. Die Zeitachse verläuft in Figur 5 nach unten. Bei der Konzentration von 1 ppm Methylenchlorid ergab sich ein Ausschlag von 75 Skalenteilen, wobei der Nullpunkt (bei Konzentration 0) bei 7 Skalenteilen liegt. Der 90 %-Ausschlag wurde bei 68,5 Skalenteilen erreicht, so daß die Differenz zwischen 100 % und 90 % bei 6,5 Skalenteilen lag. Die Anstiegszeit von 0 auf 90 % betrug 7 Minuten.

Bei der Konzentration von 0,5 ppm ergab sich ein Ausschlag von 42 Skalenteilen bei einem Nullpunkt von 7 Skalenteilen. Der 90 %-Ausschlag wurde bei 36,5 Skalenteilen in 5 Minuten erreicht. Aus dem Verlauf der Kurve 43 ist ersichtlich, daß die Meßapparatur sehr rasch auf Konzentrationsänderungen anspricht und auch den Endwert innerhalb verhältnismäßig geringer Zeit erreicht.

In Figur 6 sind die Heizeinrichtungen 23 und 31 sowie die zugehörige Beschaltung näher dargestellt. Die Heizeinrichtung 23 liegt direkt im Probedurchfluß durch die Mischkammer 16, wobei der Widerstand der Heizwendel 30 Ohm beträgt. An die elektrische Heizeinrichtung 23 wird über Leitungen 44, 45 eine Spannung von 20 V angelegt, so daß sich ein Heizstrom von 0,66 A ergibt. Die Heizleistung beträgt somit 13,3 V. Über eine Anzeigeeinrichtung 46, vorzugsweise in Form einer Anzeigelampe, die zwischen die Leitungen 44 und 45 geschaltet ist, kann angezeigt werden, ob die Heizung ein- oder ausgeschaltet ist. Die Anzeigelampe leuchtet jeweils bei eingeschalteter Heizeinrichtung 23, d.h. angelegter Eingangsspannung.

Zwischen die Leitungen 44, 45, d.h. parallel liegend zur Heizeinrichtung 23, ist eine Reihenschaltung aus der Heizeinrichtung 31 und Vorwiderständen 47, 48 geschaltet. Die Heizung für das Gassensorgehäuse hat einen Gesamtwiderstandswert von 100 Ohm, wobei 72 Ohm auf die beiden Vorwiderstände 47, 48 entfallen. Jeder Vorwiderstand 47, 48 hat jeweils einen Widerstandswert von 36 Ohm. Der Widerstandswert der Heizwendel der Heizeinrichtung 31 liegt somit bei knapp 30 Ohm. Durch die Vorwiderstände 47, 48 wird der Strom durch die Heizeinrichtung 31 verringert, verglichen mit dem Stromfluß durch die Heizeinrichtung 23. Aufgrund der Eingangsspannung von 20 V ergibt sich ein Stromfluß von 0,2 A, so daß die Leistungsaufnahme 4 Watt beträgt.

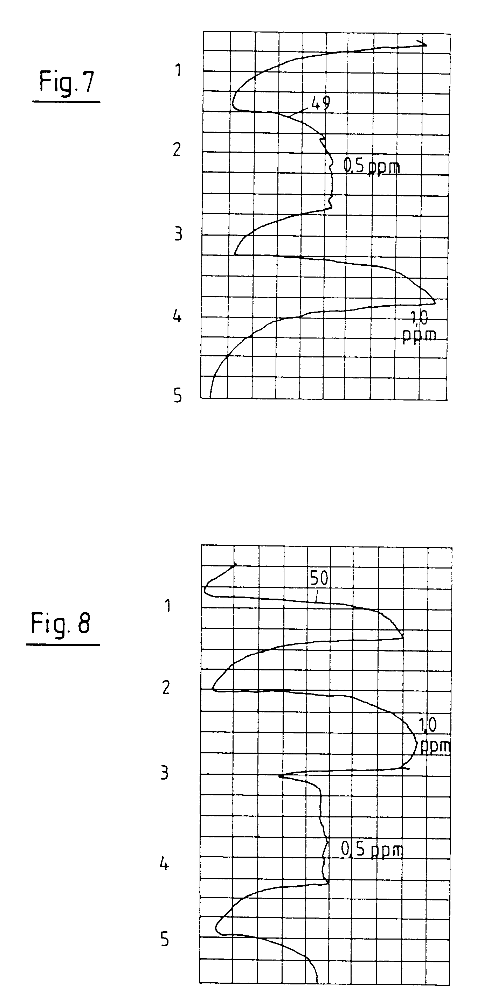

In Figur 7 ist der Verlauf der Meßkurve bei der Messung von Tetrachlorkohlenstoff bei Variation des Konzentrationswerts zwischen 0, 0,5 und 1 ppm aufgetragen. Die Meßkurve ist mit 49 bezeichnet. Die Zeitachse verläuft hierbei wie bei Figur 5 nach unten. Für die 90 %-Zeit ergaben sich folgende Werte: Bei einer Konzentration von 1 ppm lag der Vollausschlag bei 100 % bei 95 Skalenteilen und bei 90 % bei 85,5 Skalenteilen, so daß die 90 %-Differenz vom Nullpunkt 85,5 Skalenteile entsprach. Die 90 %-Zeit betrug dabei 15 Minuten.

Bei einer Konzentration von 0,5 ppm umfaßte der Vollausschlag bei 100 % 53 Skalenteile, so daß der 90 %-Wert ca. 48 Skalenteiledifferenz vom Nullpunkt entsprach. Der Nullpunkt lag bei 6 Skalenteilen. Die 90 %-Zeit betrug dabei 3 Minuten.

In Figur 8 ist eine den in Figur 5 und 7 gezeigten Kurven ähnliche Meßkurve 50 für 1,2-Dichlorethan aufgetragen, wobei die Konzentration zwischen 0, 0,5 und 1 ppm verändert wurde. Hierbei ergibt sich für die 90 %-Zeit folgendes:

Bei einer Konzentration von 1 ppm wurde bei 100 %-Vollausschlag ein Ausschlag vom 88 Skalenteilen erreicht. Die 90 %-Zeit war somit bei Erreichen von ca. 80 Skalenteilen verstrichen, wobei für die Anzeigeveränderung von 3 (bei Null-Konzentration) bis 80 Skalenteilen eine Zeitspanne von 7 Minuten benötigt wurde. Dies entsprach der 90 %-Zeit.

Bei einer Konzentration von 0,5 ppm ergab sich ein Vollausschlag von ca. 50 Skalenteilen. Die Zeit zum Erreichen eines 90 %-Ausschlags, d.h. von 45 Skalenteilen, betrug bei einem Nullpunkts-Wert von 3 Skalenteilen 7 Minuten.

Die Erfindung erlaubt somit eine sehr rasche, genaue und effektive Messung auch kleinster Verunreinigungen. Die Vorrichtung besteht dabei aus drei separaten Einheiten, nämlich dem in den Figuren 1 und 2 dargestellten Analysator, der Stromversorgung mit Temperaturregelung des Analysators, wie teilweise in Figur 6 gezeigt, und schließlich dem Auswerteteil mit Registriervorrichtung. Letzterer bewirkt die Erfassung und Auswertung der konzentrationsabhängigen Parameterveränderung des Gassensors wie etwa der Leitfähigkeitsveränderung. Da diese Auswertung bekannt ist, wird sie hier nicht näher erläutert.

## Patentansprüche

1. Vorrichtung zum Messen von Fremdstoffen, wie organische Lösungsmittel, in einer Flüssigkeit, insbesondere Wasser, die eine mit einer Heizeinrichtung (23) versehene Misch- bzw. Reaktionskammer (16), durch die die Flüssigkeit geleitet und in der die Flüssigkeit mit einem Trägergas, wie Luft od.dgl., gemischt wird, wobei die Fremdstoffe zumindest teilweise in die Gasphase überführt werden, und einen Gassensor (30) zur Messung des bei der Mischung entstehenden, aus dem Trägergas und den in die Gasphase übergegangenen Fremdstoffen zusammengesetzten Gasgemisches umfaßt, dadurch gekennzeichnet,
daß sich an die als horizontal verlaufendes, lang gestrecktes Rohr ausgebildete Mischkammer (16), eine Trennkammer (28) anschließt, in der das aus der Mischkammer (16) austretende Flüssigkeits-/Gasgemisch getrennt wird, wobei der Gassensor (30) im oberen Bereich der Trennkammer (28) angeordnet ist, die unterseitig mit einem Ablauf (33) für die Abführung der Flüssigkeit versehen ist,
daß ein Temperaturdetektor (25) zur Erfassung der Temperatur der aus der Mischkammer (16) austretenden Flüssigkeit und eine Bypass-Leitung (34) vorgesehen sind, über die das Trägergas direkt, unter Umgehung der Mischkammer (16), zum Gassensor (30) geführt wird, wobei in der Trägergas-Zuführleitung (20) ein Ventil (22) angeordnet ist, über das das Trägergas auf Durchgang zur Bypass-Leitung (34) oder auf Durchgang zur Mischkammer (16) umschaltbar ist,
daß in der Zuleitung (11) für die Flüssigkeit zur Mischkammer (16) ein Ventil (12) angeordnet ist, durch das entweder die Flüssigkeit oder eine Standardlösung (35) zu der Mischkammer (16) leitbar ist,
daß zur Förderung des Trägergases und der Flüssigkeit Dosiereinheiten (14, 21), wie z.B. Pumpen, vorgesehen sind, die synchron betrieben werden, und
daß der Gassensor (30) mit einer Heizeinrichtung (31) versehen ist, wobei die Temperatur im Bereich des Gassensors (30) auf einen Wert oberhalb der Temperatur des Gasgemisches eingeregelt wird und als Trägergas lösungsmittelfreie Luft verwendet wird.

2. Vorrichtung nach Anspruch 1, dadurch gekennzeichnet, daß das Trägergas über ein Filter, wie Aktivkohlefilter (19), angesaugt wird.

3. Vorrichtung nach einem der Ansprüche 1 und 2, dadurch gekennzeichnet, daß die Pumpen mechanisch gekoppelt sind.

4. Vorrichtung nach einem der Ansprüche 1 bis 3, dadurch gekennzeichnet, daß im Ablauf (33) eine Überlaufkappe (32) vorgesehen ist.

## Claims

1. Apparatus for determining impurities, such as organic solvents, in a liquid, particularly in water, which comprises a mixing or reacting chamber (16) provided with a heating means (23), through which the liquid is conducted and in which the liquid is intermixed with with a carrier gas, such as air or suchlike, whereby the impurities are at least in part converted into the gas phase, and a gas sensor (30) for measuring the gas mixture produced in the intermixing and composed of the carrier gas and the impurities converted into the gas phase,
characterized in that
the mixing chamber (16) constructed in the form of a horizontally proceeding, elongated pipe is followed by a separating chamber (28), wherein the mixture of liquid and gas issuing from the mixing chamber (16) is separated, in which case the gas sensor (30) is disposed within the upper area of the separating chamber (28) which, on the underside, is provided with an outlet (33) for drawing off the liquid,
in that a temperature detector (25) for detecting the temperature of the liquid issuing from the mixing chamber (16) and a bypass duct (34), through which the carrier gas is conducted direct to the gas sensor (30) while by-passing the mixing chamber (16), in which case, in the carrier gas supply line (20), a valve (20) is mounted, by means of which the carrier gas can be changed over to passage to the by-pass duct (34) or to passage to the mixing chamber (16),
in that, in the feed line (11) for the liquid to the mixing chamber (16), a valve (12) is mounted, through which either the liquid or a standard solution (35) can be conducted to the mixing chamber (16),
in that, for the delivery of the carrier gas and of the liquid, dosing units (14, 21), such as e.g. pumps, are provided which are synchronously operated, and in that, the gas sensor (30) is provided with a heating means (31), in which case, the temperature within the area of the gas sensor (30) is regulated to a value above the temperature of the gas mixture and solvent-free air is employed as carrier gas.

2. Apparatus according to Claim 1,
characterized in that
the carrier gas is drawn in through a filter, such as an activated charcoal filter.

3. Apparatus according to either Claim 1 or 2,
characterized in that
the pumps are mechanically coupled.

4. Apparatus according to any of Claims 1 to 3,
characterized in that
on overflow cap (32) is provided in the outlet (33).

## Revendications

1. Dispositif pour mesurer des impuretés, comme des solvants organiques, dans un liquide, en particulier dans de l'eau, dispositif qui comprend une chambre de mélange et/ou de réaction (16), pourvue d'un dispositif de chauffage (23), à travers laquelle le liquide est guidé et dans laquelle le liquide est mélange à un gaz porteur, comme l'air ou équivalent, les impuretés étant transportées tout au moins partiellement dans la phase gazeuse, et qui comprend un détecteur de gaz (30) pour mesurer le mélange gazeux qui se forme lors du mélange et qui est composé du gaz porteur et des impuretés transportées dans la phase gazeuse, **caractérisé en ce**
qu'une chambre de séparation (28) suit la chambre de mélange (16) qui est configurée comme un tuyau allongé dans le sens horizontal, chambre de séparation dans laquelle le mélange gazeux/liquide qui sort de la chambre de mélange (16) est séparé, le détecteur de gaz (30) étant placé dans la zone supérieure de la chambre de séparation (28) qui est pourvue sur sa face inférieure d'un écoulement (33) pour l'évacuation du liquide,
qu'un détecteur de température (25) pour détecter la température du liquide qui sort de la chambre de mélange (16) et une conduite de dérivation (34) sont prévus, conduite par laquelle le gaz porteur est guidé directement au détecteur de gaz (30) en contournant la chambre de mélange (16), une vanne (22) étant placée dans la conduite d'amenée du gaz porteur (20), vanne par laquelle le gaz porteur peut être commuté sur passage vers la conduite de dérivation (34) ou sur passage vers la chambre de mélange (16),
qu'une vanne (12) est placée dans la conduite d'amenée (11) pour le liquide vers la chambre de mélange (16), vanne par laquelle soit le liquide, soit une solution standard (35) peut être guidée vers la chambre de mélange (16),
que des unités de dosage (14, 21), qui fonctionnent de manière synchrone, comme par exemple des pompes, sont prévues pour le transport du gaz porteur et du liquide et
que le détecteur de gaz (30) est pourvu d'un dispositif de chauffage (31), la température dans la zone du détecteur de gaz (30) étant réglée à une valeur au-dessus de la température du mélange gazeux et de l'air exempt de solvant étant utilisé comme gaz porteur.

2. Dispositif selon la revendication 1, **caractérisé en ce** que le gaz porteur est aspiré par un filtre comme un filtre au charbon actif (19).

3. Dispositif selon l'une des revendications 1 et 2, **caractérisé en ce** que les pompes sont couplées mécaniquement.

4. Dispositif selon l'une des revendications 1 à 3, **caractérisé en ce** qu'un capuchon de trop-plein (32) est prévu dans l'écoulement (33).
